# EUROPEAN PATENT SPECIFICATION

(11) **EP 0 801 075 B1**
(45) Date of publication and mention of the grant of the patent: **07.07.1999**
(21) Application number: 97302416.9
(22) Date of filing: 08.04.1997
(51) Int. Cl.: C07K 9/00, A61K 38/14

(54) **Covalently linked dimers of glycopeptide antibiotics**
Kovalentgebundene Dimere von Glykopeptid-Antibiotika
Dimères liés covalemment d'antibiotiques glycopeptidiques

(30) Priority: 12.04.1996 US 15330; 25.11.1996 US 31736
(43) Date of publication of application: 15.10.1997
(73) Proprietor: ELI LILLY AND COMPANY, Indianapolis, Indiana 46285 (US)
(72) Inventor: Stack, Douglas Richard, Fishers, Indiana 46038 (US); Thompson, Richard Craig, 900 West Frankfort, Indiana 46041 (US)
(74) Representative: Hudson, Christopher Mark

(56) References cited:
- JOURNAL OF ANTIBIOTICS, vol. 49, no. 2, February 1996, TOKYO JP, pages 181-193, XP002037248 H LINSDELL ET AL.: "Dimerization of A82846B, vancomycin and ristocetin; influence on antibiotic complexation with cell wall model peptides"
- JOURNAL OF THE AMERICAN CHEMICAL SOCIETY, vol. 115, no. 1, 13 January 1993, DC US, pages 232-237, XP002037249 U GERHARD ET AL. : "The role of the sugar and chlorine substituents in the dimerization of vancomycin antibiotics"
- JOURNAL OF THE AMERICAN CHEMICAL SOCIETY, vol. 118, no. 51, 25 December 1996, DC US, pages 13107-13108, XP002037250 U N SUNDRAM ET AL.: "Novel vancomycin dimers with activity against vancomycin-resistant enterococci"

## Description

The present invention is directed to glycopeptide dimers covalently linked to one another through an amine function of an amino sugar. The invention is further directed to antibacterial methods employing, and pharmaceutical formulations comprising, such glycopeptide dimers.

Glycopeptides are a class of antibiotics; see, e.g., "Glycopeptide Antibiotics", edited by Nagarajan (Marcel Dekker, Inc., 1994). Two of them, vancomycin and teicoplanin, are sold as antibacterial products for the control of gram positive bacterial infections. Vancomycin, the earlier-discovered of the two, was used for several decades with no bacterial resistance emerging. However, in the late 1980s, resistance was detected (Lancet I, 1988, 57-88). Such resistance has increased in the years since then; see "Nosocomial Enterococci resistant to Vancomycin -- United States, 1989-1993", MMWR Morbid Mortal Wkly. Rep. (Centers for Disease Control and Prevention). Resistance can be to either or both of these antibiotics, and/or also to methicillin. Resistant organisms have become common in nosocomial settings, presenting special risks for immunocompromised persons. Resistant bacteria present a formidable challenge to society.

The present invention provides a new tool in the armamentorium for controlling resistant bacteria.

The present invention is directed to glycopeptide dimers which are covalently linked through an amine function of an amino sugar. The identity of the glycopeptide is not critical, except that it comprises a modifiable amine on a sugar. Preferred glycopeptides are those of the vancomycin type, also known as dalbaheptides (J.Antibiotics, Dec., 1989, page 1892).

Representative glycopeptides include:
vancomycin,
A82846A,
A82846B,
A82846C,
PA-42867-A,
PA-42867-C,
PA-42867-D,
A83850A,
A83850B,
actinoidin,
avoparcin,
galacardin,
helevecardin, and
M47767.

The linking group which functions to covalently link the two glycopeptide units is similarly not critical. The present dimers are most conveniently prepared by the reaction of the glycopeptide and a bisaldehyde, followed by reduction. Therefore, the linking group is the chemical unit internal to the aldehyde groups of any bisaldehyde.

In one embodiment, the present invention is directed to specific dimer compounds defined by Formula I: 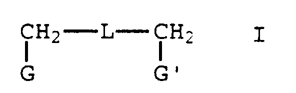 In the above formulae, each of G and G' is independently selected from the group consisting of deshydrovancomycin of the formula: 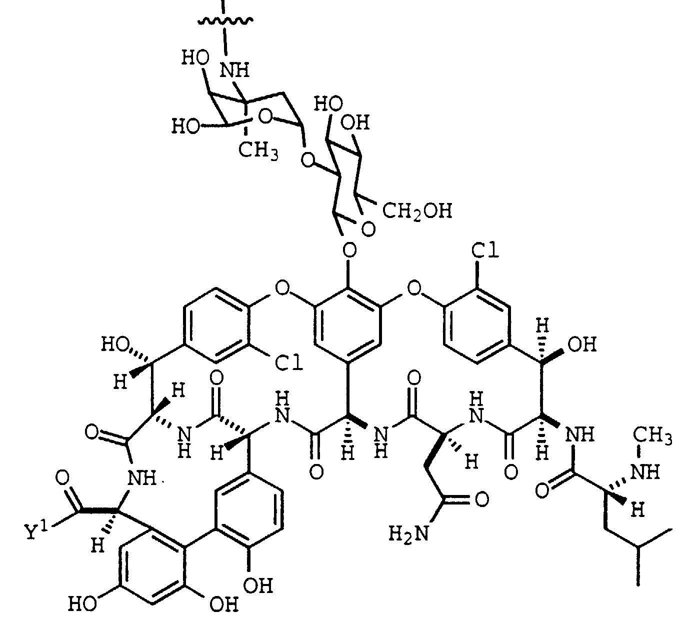 and deshydroA82846B of the formula: 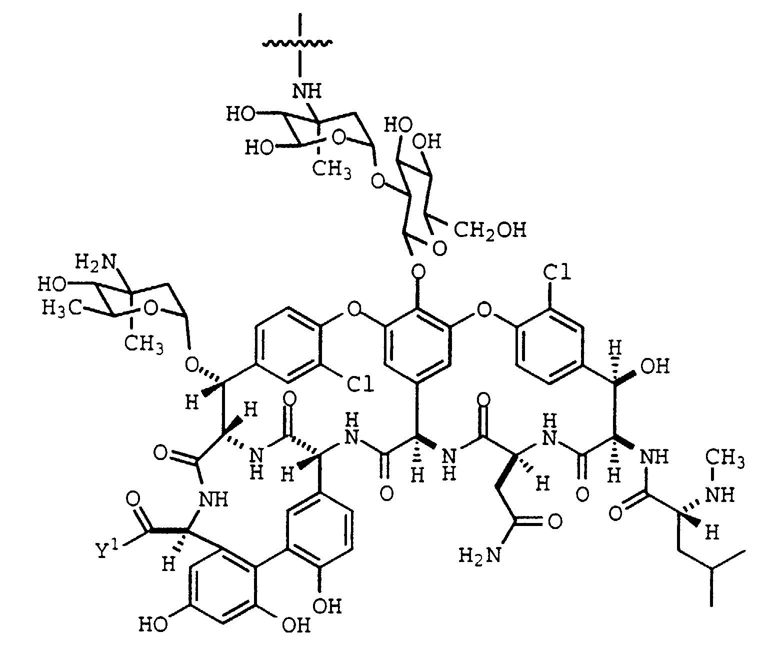 wherein Y¹ is OH or 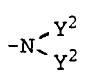 and Y² is defined as follows:
(1) each Y² independently represents
   hydrogen,
   alkyl of C₁-C₁₀,
   cycloalkyl of C₅-C₆.
   cycloalkenyl of C₅-C₆,
   naphthyl,
   biphenylyl,
   radical of the formula -Y³-(Y⁴)0, 1, or 2, wherein Y³ is loweralkyl of C₁-C₆ optionally substituted by from one to three substituents, each of which is independently selected from the group consisting of halo, nitro, cyano, alkoxy, haloalkyl, and haloalkoxy; and Y⁴ is 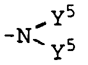 wherein Y⁵ is independently hydrogen or loweralkyl of C₁-C₄, or Y⁴ is phenyl or phenyl substituted with from one to three substituents, each of which is independently
   halo,
   nitro,
   loweralkyl of C₁-C₄,
   cycloalkyl of C₅-C₆,
   loweralkoxy of C₁-C₄,
   haloloweralkyl of C₁-C₄, or
   haloloweralkoxy of C₁-C₄; or
(2) one Y² is hydrogen and the other Y² is (2-furanon-3-yl); or
(3) both Y²s are taken together with the nitrogen and constitute a five- to seven-membered heterocyclic ring optionally containing in addition to the indicated nitrogen atom one additional hetero ring atom which is nitrogen, oxygen, or sulfur, and which heterocyclic radical can be unsubstituted or substituted with from one or two substituents, each of which is loweralkyl of C₁-C₂, loweralkoxy of C₁-C₂, phenyl, benzyl, or C₁-C₆-alkanoyl; and L is a divalent linking radical of the formula A: 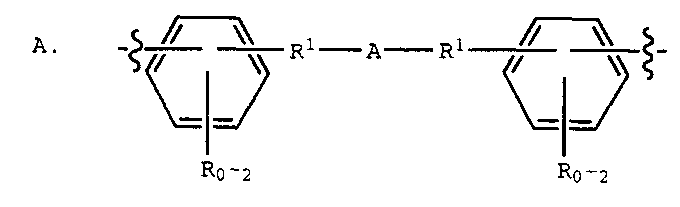 wherein A is:
   alkylene of C₁-C₁₆,
   (alkylene of C₁-C₄-X')_{q}-alkylene of C₁-C₄, wherein q is 1-3,
   alkylene of 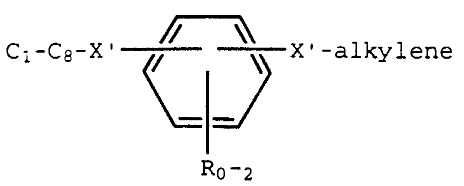 of C₁-C₈;
   alkylene of 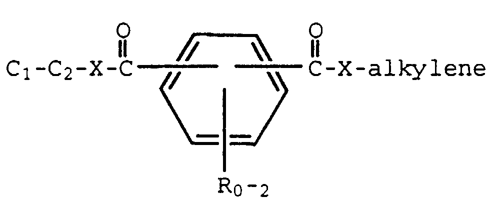 of C₁-C₂, or
   alkylene of 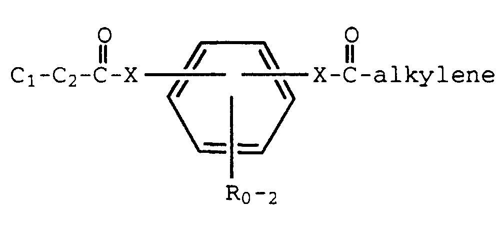 of C₁-C₂;
each R¹ is independently
CH_{2,}
O,
S, 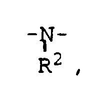 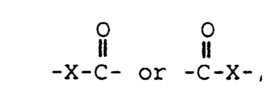 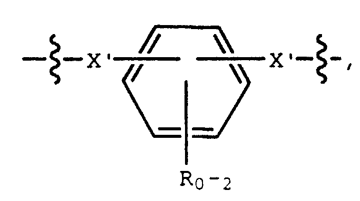 or 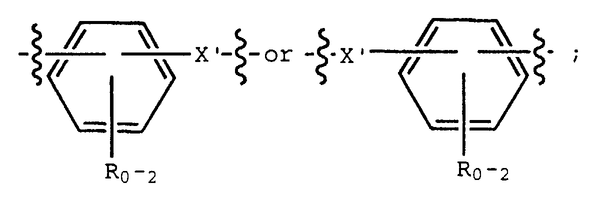 wherein each R independently represents halo, loweralkyl of C₁-C₆, loweralkoxy of C₁-C₆, phenyl, or phenyl substituted by from 1 to 2 substituents, each of which is independently halo, loweralkyl of C₁-C₆, or loweralkoxy of C₁-C₆; each X is independently -O- or 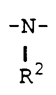 wherein R² is H or loweralkyl of C₁-C₄; and each X' is independently -O-, -S-, or 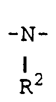 wherein
R² is as defined above; or L is a divalent linking radical of the formula B:

B. -alkylene of C₁-C₈-R³-X"-R³-alkylene of C₁-C₈-

wherein X" represents alkylene of C₁-C₄ or a phenylene of the formula 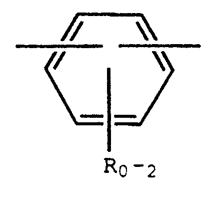 wherein R is as defined above; and each R³ is independently CH₂ or O. Salts of the foregoing dimers can be used.

In compounds of Formula I, the glycopeptide units, G and G', may be identical or different. Any "alkylene" of C₂ or higher can be straight chain or branched.

Certain compounds are preferred. Symmetrical compounds (G=G' and/or both R¹ are identical), are preferred for their more efficient synthesis.

Antibacterial activity is enhanced by employing preferred "L" groups. Preferences include the following, individually and in any combination:
L = a linking radical of formula A
L = a linking radical of formula B wherein the carbon attached to -CH₂-G or to -CH₂-G' is branched
R¹ = O
A = alkylene of C₁-C₁₆, especially straight-chain and especially C₆-C₁₂;
A = (alkylene of C₁-C₄-X')_{q}-alkylene of C₁-C₄, especially wherein X'=O; the alkylene is -(CH₂)₂-;
and q=2;
R = phenyl and substituted phenyl, especially chlorophenyl; and especially when R has this value on a phenyl ring within "A".
Other preferences will be apparent from the further teachings herein.

Representative dimers of Formula I are set forth in following TABLE 1.

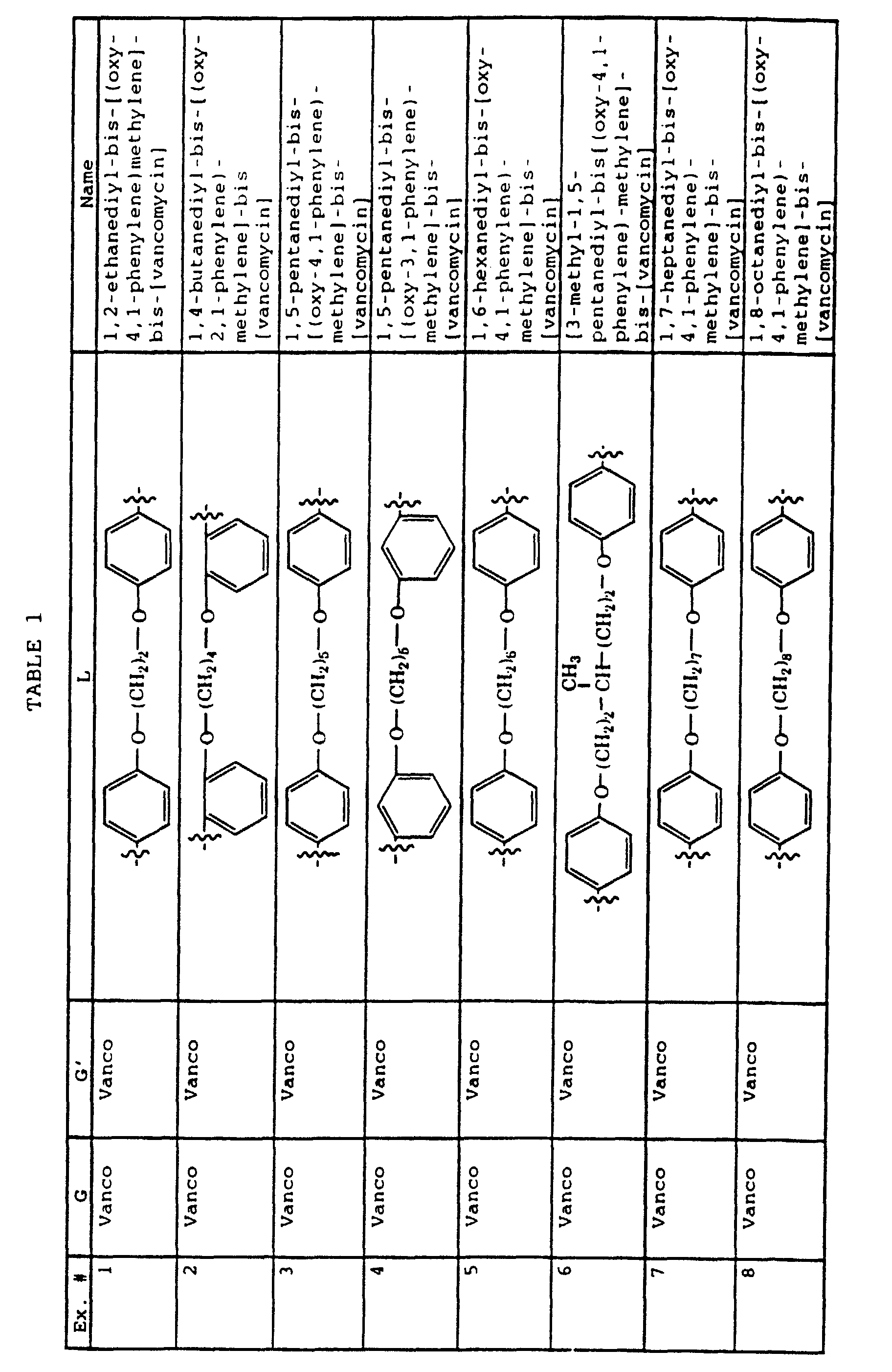

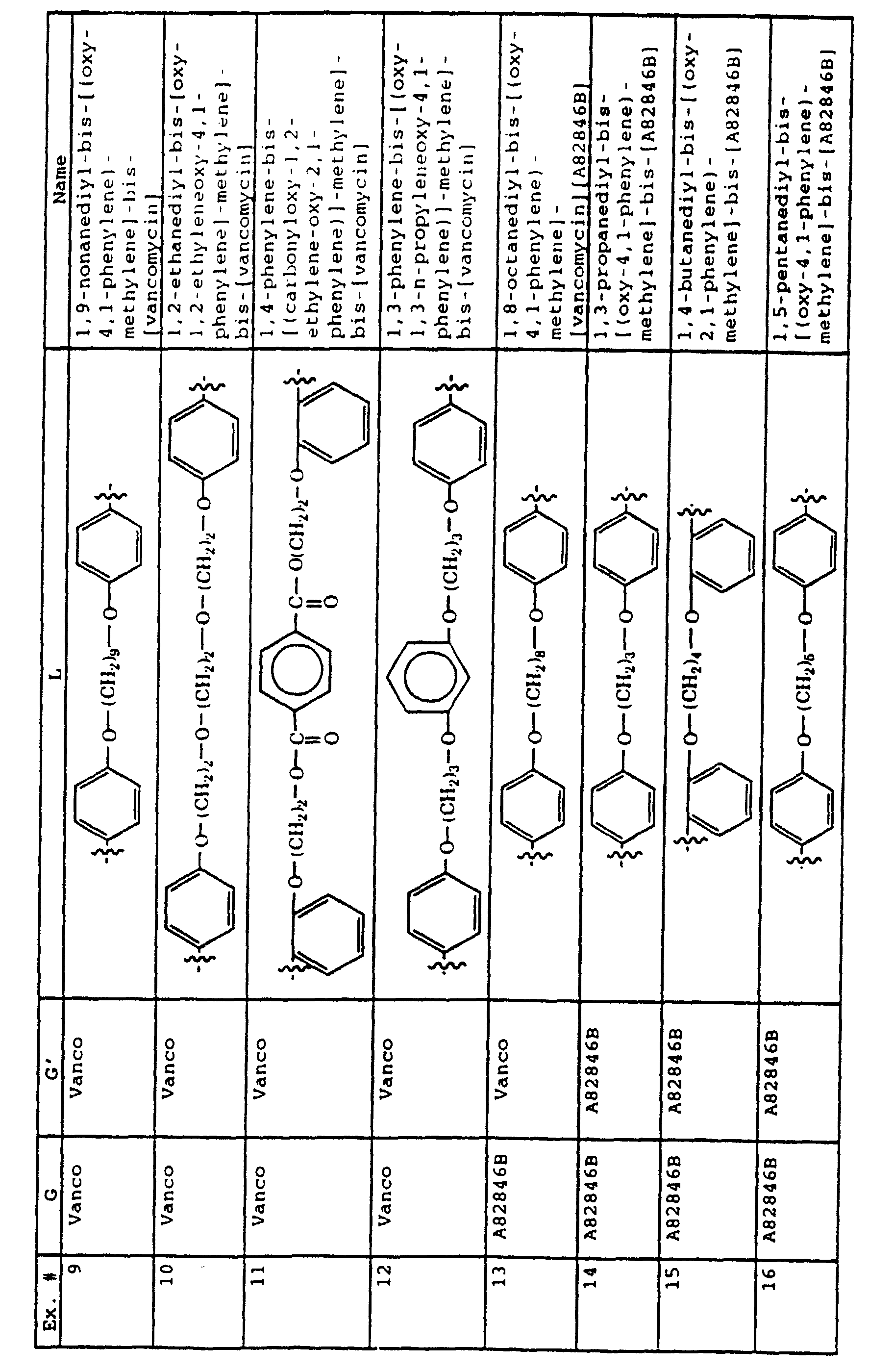

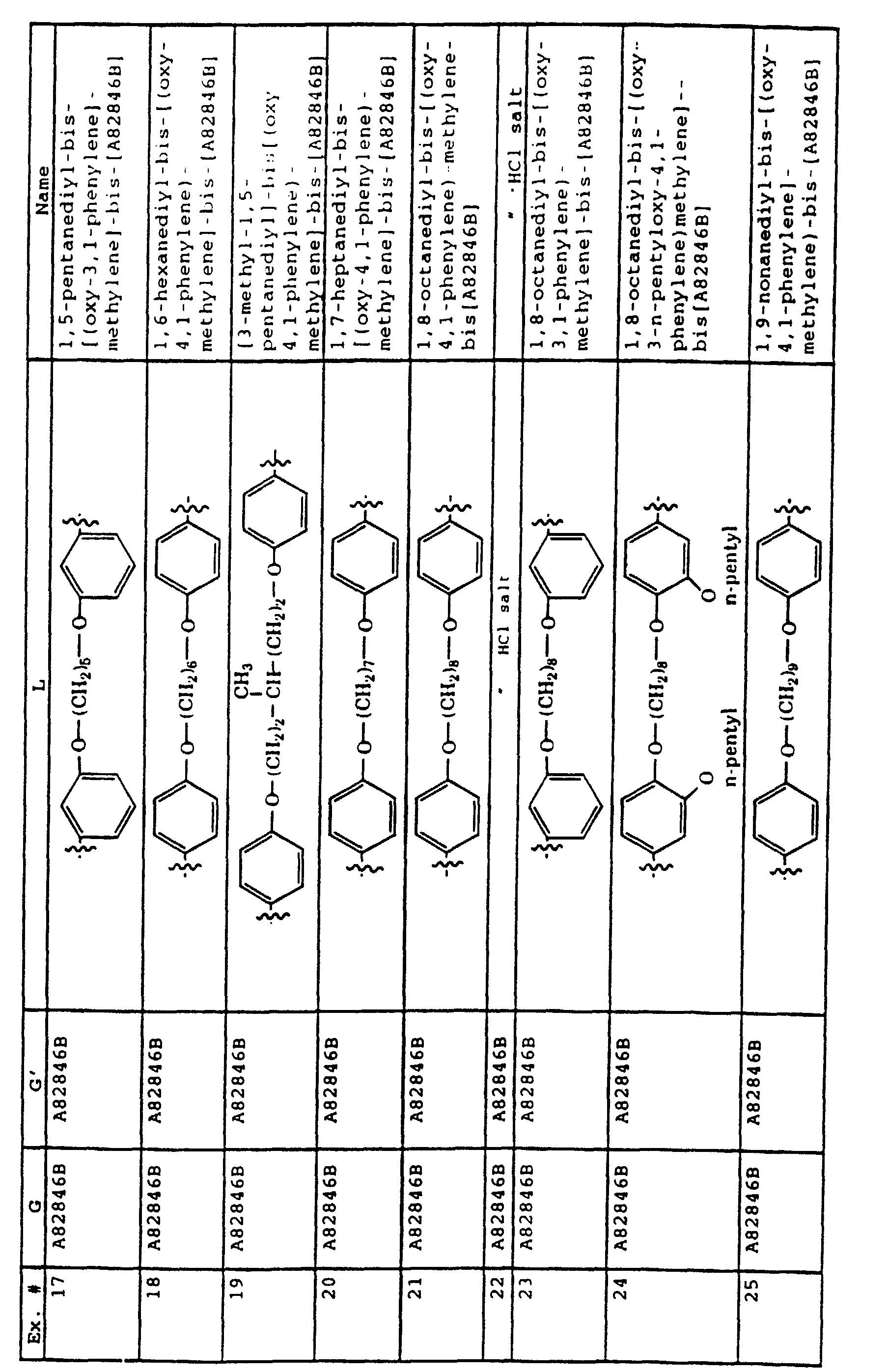

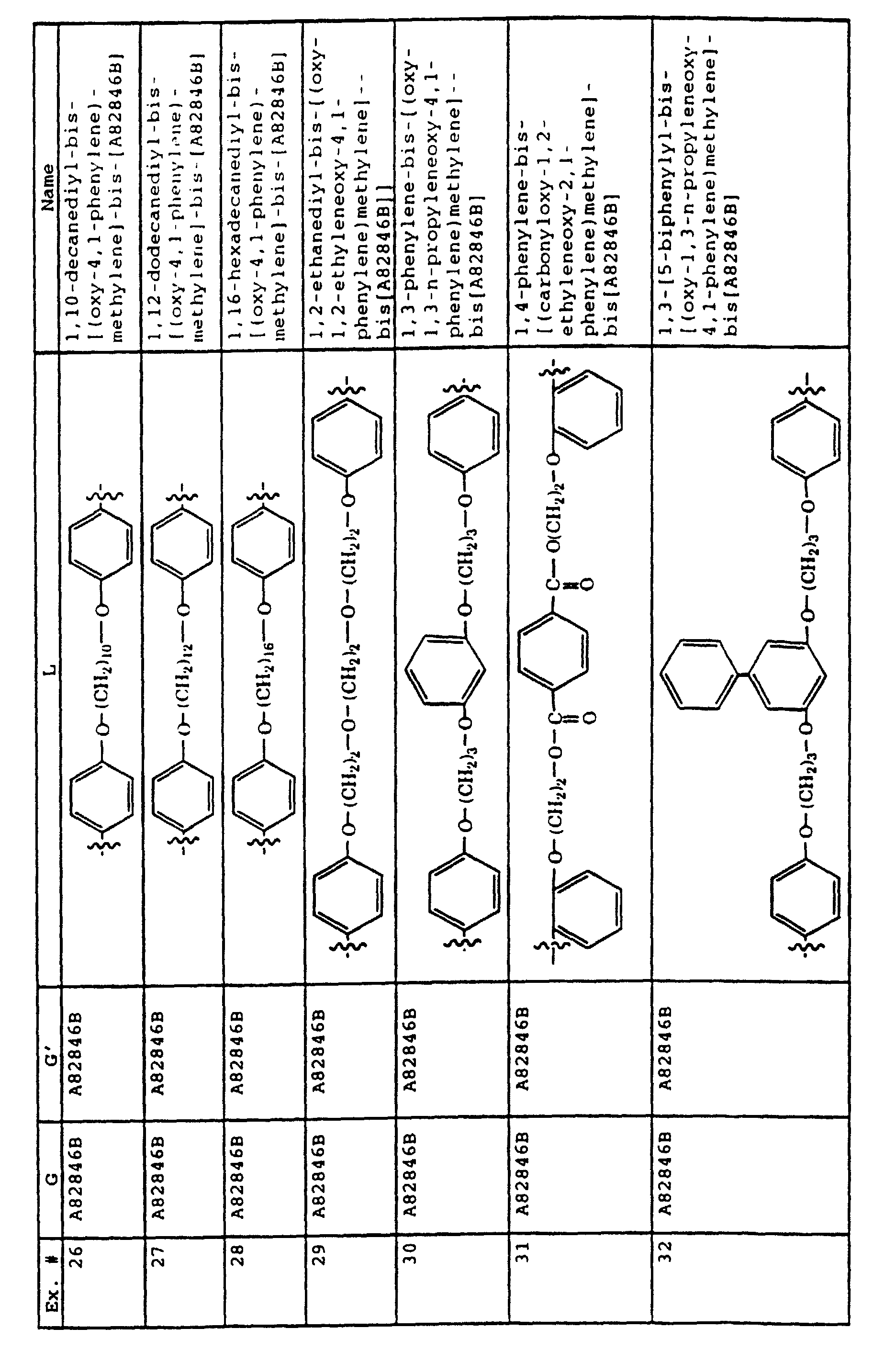

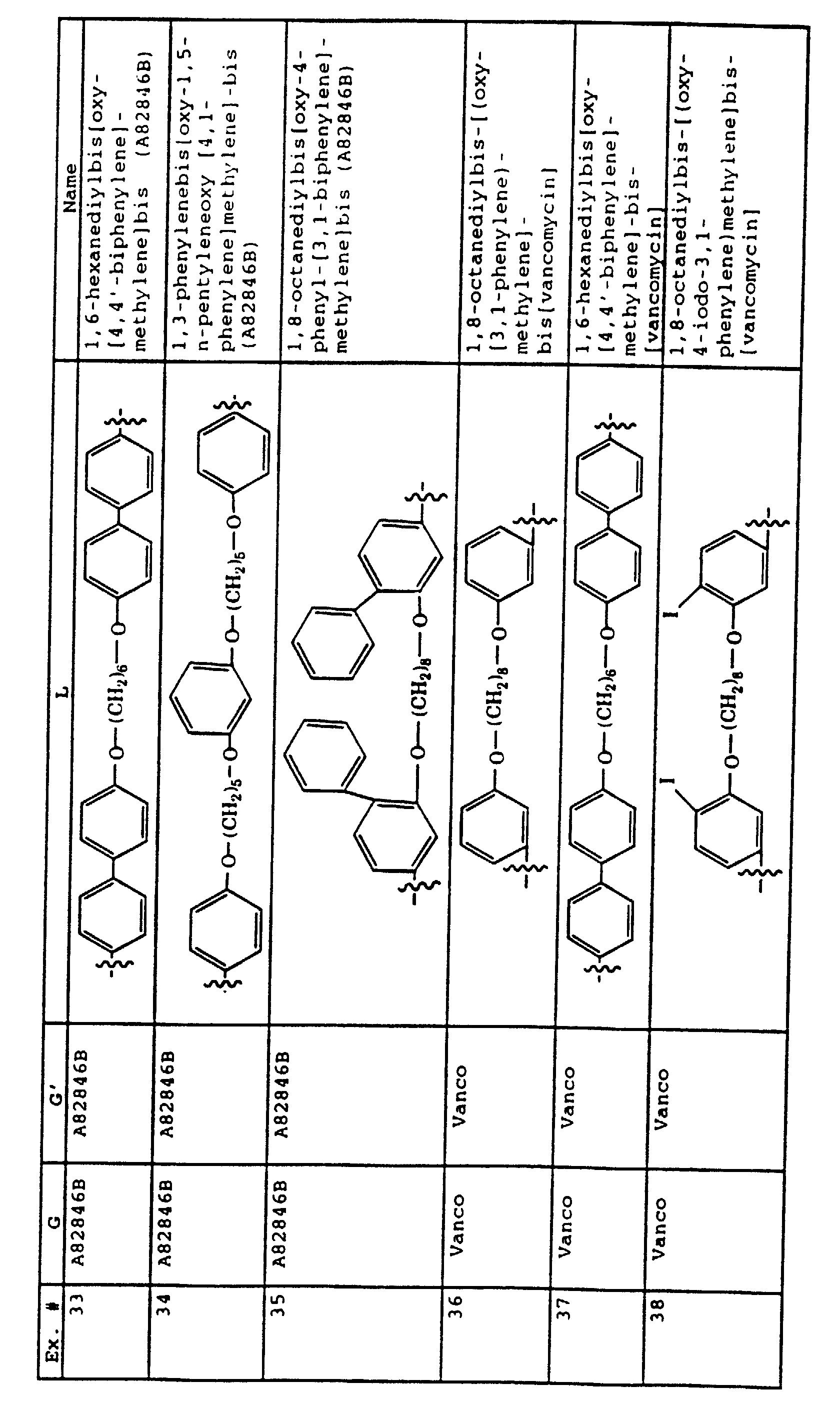

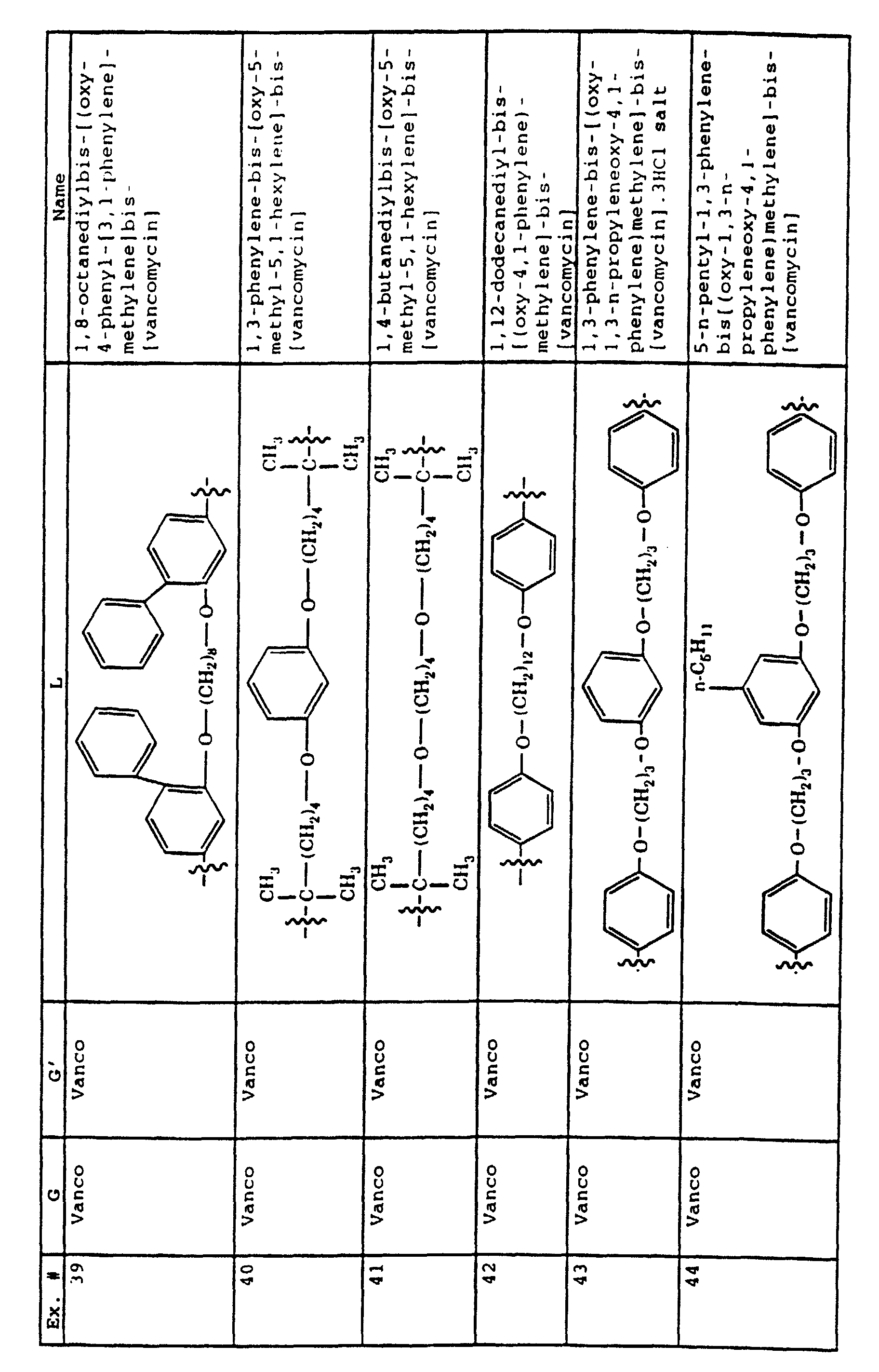

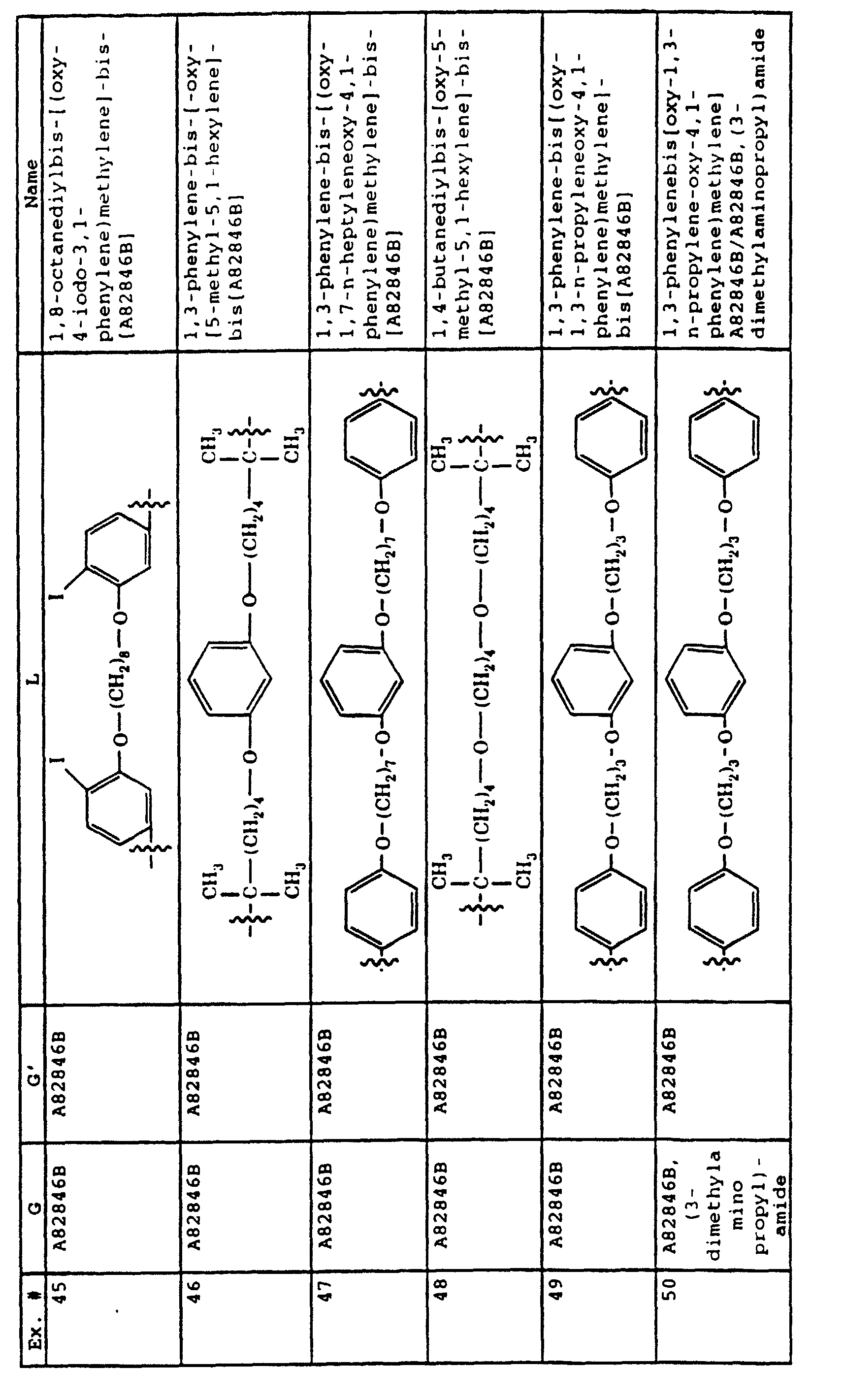

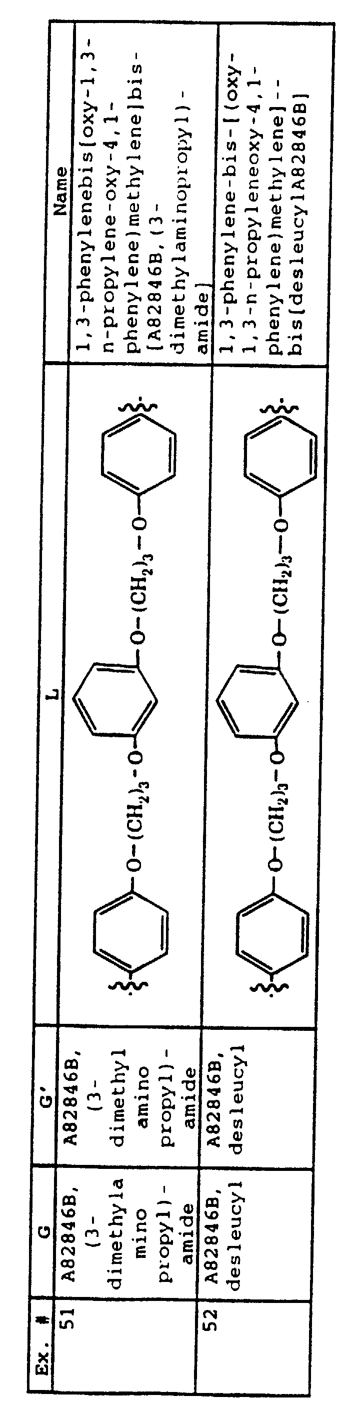

The dimers of the present invention are prepared by reacting a glycopeptide with a bisaldehyde to form an intermediate Schiff base, which is subsequently reduced to obtain the dimers.

Many bisaldehydes are known compounds. They can be prepared by techniques known to those skilled in the art, per various references;
J. Org. Chem., **26**, 474, (1961)
J. Het. Chem., **27**, 1007 (1990)
J.A.C.S., **73**, 1872 (1951)
J.A.C.S., **109**, 2260 (1987)
J. Chem. Soc. Perkin I, 189 (1983)
Syn. Comm., **18(12)**, 1379 (1988)
Chem. Letters, 587 (1995)
Macromolecules, 6045 (1992);
J. Chem. Soc. Chem. Comm. 1463 (1991)
J. Polym. Sci. Part A. Polymer Chem. 31(12) 2899 (1993)
J. Chem. Res. Synop. (8), 296 (1994)
Farmco Ed. Sci. 15, 468 (1960)
Makromol. Chem. 191 (4) 815 (1990)
J. Polym. Sci. Part A. Polymer Chem. 29(3) 361 (1991)
Makromol. Chem. 65, 54 (1963)

The reaction of bisaldehyde with glycopeptide is carried out in accordance with prior art condensations of amine and aldehyde to form Schiff bases, and their subsequent reduction.

Thus, the present condensation is typically conducted in a polar solvent, such as dimethylformamide or methanol, or a mixture of polar solvents. The reaction goes forward over a range of temperatures, such as from 25°C to 100°C, but is preferably conducted at temperatures of about 60°C to 70°C. The reaction is preferably conducted under an inert atmosphere, such as nitrogen or argon. The reaction requires two molecular proportions of glycopeptide and one molecular proportion of bisaldehyde.

The reaction yields a Schiff base of the formula

G=CH-L-CH=G'.

The Schiff base is subsequently reduced. Preferably, the reduction is conducted in the same reaction mixture in a polar solvent, and employing a chemical reducing agent. Metal borohydrides, such as sodium borohydride and sodium cyanoborohydride are preferred. The reaction goes forward over a range of temperatures, such as from about 25°C to about 100°C; preferably, the reaction is conducted at about 60°C to 70°C.

The product, or mixture of products, can be isolated and purified if desired in a conventional manner, such as by HPLC. Characterization of products is best accomplished by Fast Atom Bombardment Mass Spectroscopy (FAB•MS).

In addition to the foregoing synthetic route, compounds of the present invention can be prepared in an alternate route. In this alternate route, a dimer is prepared by the foregoing synthetic route, and further changes to the structure of the glycopeptide are made subsequently. This approach to synthesizing the present dimers is illustrated by Preparations 6-8 below. In Preparations 6-7, a dimer of the present invention is reacted with an amine to convert the acid of the glycopeptide to an amide 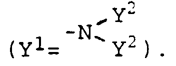 In Preparation 8, a dimer of the present invention is subjected to Edman degradation to obtain the corresponding desleucyl dimer. Other modifications of the glycopeptide portion of a dimer can likewise be made. Techniques for such modifications are known to those skilled in the art; see Glycopeptide Antibiotics, supra, and references cited therein. This volume is incorporated herein by reference.

When it is desired to employ a salt, a compound of Formula I can be reacted with a mineral or organic acid or an inorganic base, in techniques well known to those skilled in the art. Pharmaceutically-acceptable salts are preferred.

The following examples report preparations of illustrative dimers.

The HPLC procedures reported in these examples were as follows:

Analytical ("Conditions A"): Reactions were monitored by analytical HPLC using a Waters µBondapak C₁₈ column (3.9x300 mm) and UV detection at 280 nm. Elution was accomplished with a linear gradient of 5% CH₃CN - 95% buffer to 80% CH₃CN - 20% buffer over 30 minutes. The buffer used was 0.5% triethylamine in water, adjusted to pH 3 with H₃PO₄.

Preparative ("Conditions B"): Crude reaction mixtures were purified by preparative HPLC using a Waters C₁₈ Nova-Pak column (40x300 mm) and UV detection at 280 nm. Elution was accomplished with a linear gradient of 5% CH₃CN - 95% buffer to 80% CH₃CN - 20% buffer over 30 minutes. The buffer used was 0.5% triethylamine in water, adjusted to pH 3 with H₃PO₄. The desired fractions were subsequently desalted with a Waters C₁₈ Sep-Pak (35 cc) followed by lyophilization. Alternatively, a buffer containing 0.1% TFA in H₂O can be used, in which case the TFA salt is obtained directly after lyophilization.

Compounds were desalted as follows. A Waters Sep-Pak cartridge was pre-wet with methanol (2-3 column volumes) then conditioned with water (2-3 column volumes). The sample, dissolved in a minimum volume of water, was loaded onto the Sep-Pak column which was then washed with water (2-3 column volumes) to remove the unwanted salts. The product was then eluted with an appropriate solvent system, typically 1:1 CH₃CN/H₂O, CH₃CN, and/or methanol. The organic solvent component was removed *in vacuo* and the resulting aqueous solution lyophilized to give the final product.

### Preparation 1:

### Synthesis of Example 5, 1,6-hexanediylbis[(oxy-4,1-phenylene) methylene]bis[vancomycin]

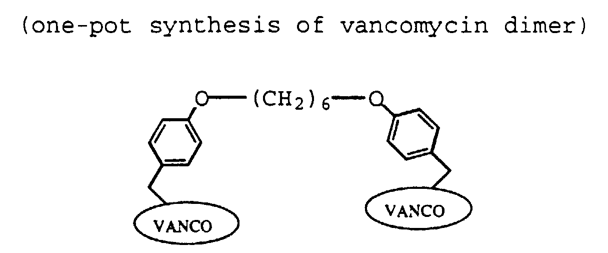

A dry 100 mL round bottom flask was charged with vancomycin•HC1 (250 mg, 0.168 mmol.), and 1,6-bis(4'-formylphenoxy)-n-hexane(101 mg, 0.310 mmol.). Anhydrous DMF (6 mL) was added to the flask and the resulting mixture was stirred under N₂ and heated to 70°C. After 3.5 hours, sodium cyanoborohydride (80 mg, 1.3 mmol.) was added in one portion, and the reaction mixture was maintained at 70°C for one additional hour. The reaction mixture was cooled, and stored at 0°C overnight.

The reaction mixture was then concentrated in vacuo to give a residue which was re-dissolved in 1:1 H₂0:CH₃CN (5 mL) and HOAc (0.5 mL). The resulting solution was purified by preparatory HPLC (conditions B). The desired fractions, as determined by analytical HPLC (conditions A), were concentrated in vacuo to ∼ 1.5 mL, and desalted. After lyophilization, 1,6-hexanediylbis[(oxy-4,1-phenylene)methylene]bis[vancomycin] was obtained (24.3 mg, 0.008 mmol., 10.0 % yield) as a white powder.
HPLC (conditions A) retention time: 13.6 min.
FABMS shows peak of (M+6H) at 3195.

### Preparation 2:

### Synthesis of Example 25, 1,9-nonanediylbis[(oxy-4,1-phenylene)methylene]bis[A82846B]

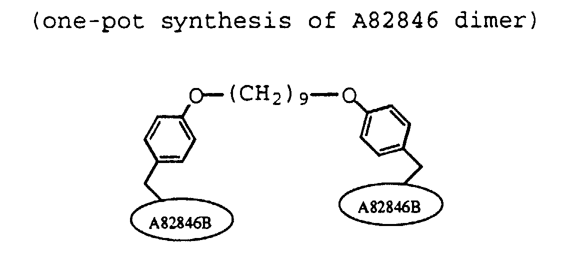

A dry 100 mL round bottom flask was charged with A82846B·tri-acetate salt (278 mg, 0.157 mmol.), and 1,9-bis-(4'-formylphenoxy)-n-nonane (103.7 mg, 0.282 mmol.) . Anhydrous DMF (15 mL) and anhydrous MeOH (15 mL) were added to the flask and the resulting mixture was stirred under N₂ and heated to 70°C. After 3.5 hours, sodium cyanoborohydride (68 mg, 1.08 mmol.) was added in one portion, and the reaction mixture was maintained at 70°C for one additional hour.

The reaction mixture was then concentrated in vacuo to give a residue which was re-dissolved in 1:1 H₂0:CH₃CN (5 mL) and HOAc (0.5 mL). The resulting solution was purified by preparatory HPLC (conditions B). The desired fractions, as determined by analytical HPLC (conditions A), were concentrated in vacuo to ∼ 1.5 mL, and desalted. After lyophilization, 1,9-nonanediylbis[(oxy-4,1-phenylene)methylene]bis[epivancomycin] was obtained (25.7 mg, 0.007 mmol., 9.3 % yield) as a white powder.
HPLC (conditions A) retention time: 14.9 min.
FABMS shows peak of (M+5H)at 3522.

### Preparation 3:

### Synthesis of Example 13, 1,8-octanediylbis[(oxy-4.1-phenylene)methylene][vancomycin] [A82846B]

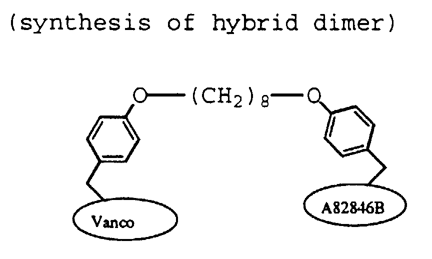

A dry round bottom flask was charged with vanconycin•HCl (75 mg, 0.052 mmol.), and N⁴-(4-(8-(p-formylphenoxy)-n-octyloxy)benzyl)A82846B (50 mg, 0.026 mmol.) (see Preparation 4). Anhydrous DMF (6 mL) was added to the flask and the resulting mixture was stirred under N₂ and heated to 70°C. After 5 hours, sodium cyanoborohydride (59 mg, 0.93 mmol.) was added in one portion, and the reaction mixture was maintained at 70°C for one additional hour. The reaction mixture was cooled, and stored at 0°C overnight.

The reaction mixture was then concentrated in vacuo to give a residue which was re-dissolved in 1:1 H₂0:CH₃CN (5 mL) and HOAc (0.5 mL). The resulting solution was purified by preparatory HPLC (conditions B). The desired fractions, as determined by analytical HPLC (conditions A), were concentrated in vacuo to ∼ 1.5 mL, and desalted. After lyophilization, 1,8-octanediylbis[(oxy-4,1-phenylene)methylene][vancomycin][A82846B] was obtained (5.2 mg, 0.002 mmol., 7.6 % yield) as a white powder.
HPLC (conditions A) retention time: 14.5 min.
FABMS shows peak of (M+6H) at 3364.

### Preparations 4 & 5:

### Synthesis of Example 47, N⁴-(4-(8-(p-formylphenoxy)-n-octyloxy)benzyl)A82846B, and Example 21, 1,8-octanediylbis[(oxy-4,1-phenylene) methylene]bis[A82846B]

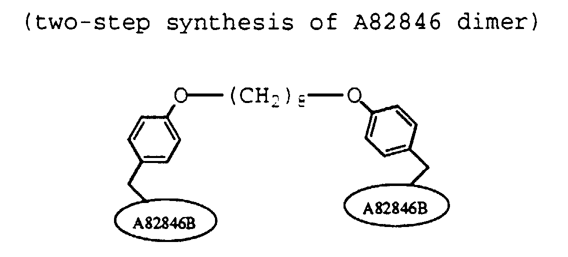

A dry flask was charged with A82846B·tri-acetate salt (5.0 g, 0.003 mol.), and 1,8-bis(4'-formylphenoxy)-n-octane (1.93 g, 0.006 mol.). Anhydrous DMF (300 mL) and anhydrous MeOH (300 mL) were added to the flask and the resulting mixture was stirred under N₂ and heated to 70°C. After 3.75 hours, sodium cyanoborohydride (0.76 g, 0.012 mol.) was added in one portion, and the reaction mixture was maintained at 70°C for one additional hour. The reaction was cooled and stored at 0°C overnight.

The reaction mixture was then concentrated in vacuo to give a residue which was re-dissolved in 1:1 H₂O:CH₃CN (200 mL) and HOAc (5 mL). The resulting solution was purified by preparatory HPLC (conditions B). The desired fractions, as determined by analytical HPLC (conditions A), were concentrated in vacuo to - 1.5 mL, and desalted. After lyophilization, N⁴-(4-(8-(p-formylphenoxy)-n-octyloxy)benzyl)A82846B was obtained (387.4 mg, 0.2 mmol., 6.6 % yield) as a white powder.
HPLC (conditions A) retention time: 19.9 min.
FABMS shows peak of (M+3H)at 1932.

A dry flask was charged with N4-(4-(8-(p-formylphenoxy)-n-octyloxy)benzyl)A82846B (20.0 mg, 0.01 mmol), and A82846B (32.9 mg, 0.021 mmol). Anhydrous DMF (3 mL) and anhydrous MeOH (3 mL) were added to the flask and the resulting mixture was stirred under N₂ and heated to 70°C. After 2 hours, sodium cyanoborohydride (5.0 mg, 0.079 mmol) was added in one portion, and the reaction mixture stirred an additional 0.25 hours.

The reaction mixture was then concentrated in vacuo to give a residue which was redissolved in 1:1 H₂O:CH₃CN (5 mL). The resulting solution was purified by preparatory HPLC (conditions D). The desired fraction, as determined by analytical HPLC (conditions A), were concentrated in vacuo to -1.5 mL, and desalted. After lyophilization, 1,8-octanediylbis[(oxy-4,1-phenylene)methylene]bis[A82846B] was obtained (3.0 mg, 0.001 mmol, 8.6 % yield) as a white powder.
HPLC (conditions A) retention time: 13.6 min.
FABMS shows peak of (M+5H) at 3508.

### Preparations 6 & 7:

### Synthesis of Example 50, 1,3-phenylenebis[oxy-1,3-n-propylene-oxy-4,1-phenylene)methylene]A82846B/A82846B, (3-dimethylaminopropyl)amide, and Example 51, 1,3-phenylenebis[oxy-1,3-n-propylene-oxy-4,1-phenylene)methylene]bis[A82846B, (3-dimethylaminopropyl)amide]

A dry round bottom flask was charged with 1,3-phenylenebis-[(oxy-1,3-n-propyleneoxy-4,1-phenylene)methylene]-bis[A82846B] (50.0 mg, 0.014 mmol) and 1 mL DMSO. PyBOP (14.5 mg, 0.028 mmol) and 3-dimethylaminopropylamine (2.8 mg, 0.028 mmol) were added and the reaction was stirred at room temperature under nitrogen for one hour. The reaction mixture was then concentrated in vacuo to give a residue which was re-dissolved in 1:1 H₂O:CH₃CN (5 mL). The resulting solution was purified by preparatory HPLC (conditions B). The desired fractions, as determined by analytical HPLC (conditions A) were concentrated in vacuo to - 1.5 mL, and desalted as in previous examples. After lyopholization 1,3-phenylenebis[oxy-1,3-n-propylene-oxy-4,1-phenylene)methylene]bis[A82846B, (3-dimethylaminopropyl)amide] (6.9 mg, 13.1% yield) and 1,3-phenylenebis[oxy-1,3-n-propylene-oxy-4,1-phenylene)methylene]A82846B/A82846B,(3-dimethylaminopropyl)amide (6.6 mg, 12.8% yield) were obtained as white powders.
1,3-phenylenebis[oxy-1,3-n-propylene-oxy-4,1-phenylene)methylene]bis[A82846B, (3-dimethylaminopropyl)amide]
HPLC (conditions A) retention time: 13.2 min.
FABMS shows peak of (M+9H) at 3761.
1,3-phenylenebis[oxy-1,3-n-propylene-oxy-4,1-phenylene)methylene]A82846B/A82846B, (3-dimethylaminopropyl)amide HPLC (conditions A) retention time: 13.7 min.
FABMS shows peak of (M+6H) at 3674.

### Preparation 8:

### Synthesis of Example 52, 1,3-phenylene-bis-[(oxy-1,3-n-propyleneoxy-4,1-phenylene)methylene]bis[desleucylA82846B]

1,3-phenylene-bis-[(oxy-1,3-n-propyleneoxy-4,1-phenylene)methylene]-bis[A82846B] (165.8 mg, 0.0462 mmol) was dissolved in 15 mL H₂O - pyridine (1:1 v/v) and treated with phenyl isothiocyanate (30 µl, 0.25 mmol). The resulting mixture was stirred at room temperature for 1.5 hours at which time HPLC analysis (conditions A) indicated complete consumption of the starting material. The reaction mixture was concentrated *in vacuo* to give the crude bisthiourea derivative as a white powder.

The crude thiourea intermediate was suspended in 15 mL CH₂Cl₂, cooled to 0°C, then treated with trifluoroacetic acid (0.20 mL). After 1 hour the reaction mixture was warmed to room temperature and stirred an additional 1 hour. An additional 0.20 mL trifluoroacetic acid was added and the mixture was stirred at room temperature for 3 hours. The solvent was removed *in vacuo* and the crude product was purified by preparative HPLC (conditions B). The desired fractions, as determined by analytical HPLC (conditions A) were concentrated *in vacuo* to ∼ 1.5 mL, and desalted as in previous examples to give 10.2 mg (7% yield) of 1,3-phenylene-bis-[(oxy-1,3-n-propyleneoxy-4,1-phenylene)methylene]-bis[desleucylA82846B] as a white powder.
FAB-MS: obtained 3333 (M+4)
HPLC retention time (conditions A): 15.1 min.

Details concerning the synthesis of all of the compounds of TABLE 1, as well as identifying characteristics, are presented in TABLE 2.

**TABLE 2**

| Ex. # | Aldehyde | HPLC * Retention Minutes | % yield | FAB•MS M/Z | M+x H |
|---|---|---|---|---|---|
| 1 | 1,2-bis(4-formylphenoxy)-n-ethane | 11.5 | 1.86 | 3136 | 3 |
| 2 | 1,4-bis(2-formylphenoxy)-n-butane | 11.8 | 0.79 | 3165 | 4 |
| 3 | 1,5-bis(4-formylphenoxy)-n-pentane | 12.9 | 5.42 | 3178 | 4 |
| 4 | 1,5-bis(3-formylphenoxy)-n-pentane | 13.0 | 4.08 | 3179 | 4 |
| 5 | 1,6-bis(4-formylphenoxy)-n-hexane | 13.6 | 9.05 | 3195 | 6 |
| 6 | 3-methyl-1,5-bis(4-formylphenoxy)-n-pentane | 13.5 | 4.54 | 3193 | 4 |
| 7 | 1,7-bis(4-formylphenoxy)-n-heptane | 14.7 | 5.00 | 3207 | 5 |
| 8 | 1,8-bis(4-formylphenoxy)-n-octane | 15.5 | 3.91 | 3219 | 2 |
| 9 | 1,9-bis(4-formylphenoxy)-n-nonane | 16.4 | 4.41 | 3235 | 4 |
| 10 | 1,2-bis(2-(4-formylphenoxy)-ethoxy)ethane | 12.3 | 1.89 | 3226 | 4 |
| 11 | 1,4-bis(2-(p-formylphenoxy)ethoxy)carbonylbenzene | 13.0 | 10.50 | 3331 | 6 |
| 12 | 1,3-bis(3-(p-formylphenoxy)-n-propyloxy)benzene | 15.5 | 3.10 | 3300 | 4 |
| 13 | 1,8-bis(4-formylphenoxy)-n-octane | 14.5 | 5.95 | 3364 | 4 |
| 14 | 1,3-bis(4-formylphenoxy)propane | 9.4 | 14.29 | 3436 | 4 |
| 15 | 1,4-bis(2-formylphenoxy)-n-butane | 10.2 | 5.91 | 3452 | 5 |
| 16 | 1,5-bis(4-formylphenoxy)-n-pentane | 10.4 | 3.86 | 3466 | 5 |
| 17 | 1,5-bis(3-formylphenoxy)-n-pentane | 11.3 | 22.41 | 3465 | 4 |
| 18 | 1,6-bis(4-formylphenoxy)-n-hexane | 11.3 | 5.46 | 3478 | 4 |
| 19 | 3-methyl-1,5-bis(4-formylphenoxy)-n-pentane | 11.3 | 8.14 | 3479 | 4 |
| 20 | 1,7-bis(4-formylphenoxy)-n-heptane | 12.5 | 5.73 | 3494 | 5 |
| 21 | 1,8-bis(4-formylphenoxy)-n-octane | 14.0 | 13.31 | 3508 | 5 |
| 23 | 1,8-bis(3-formylphenoxy)-n-octane | 14.4 | 21.23 | 3508 | 5 |
| 24 | 1,8-bis(4-formyl-2-n-pentyloxyphenoxy)-n-octane | 20.8 | 16.91 | 3680 | 5 |
| 25 | 1,9-bis(4-formylphenoxy)-n-nonane | 14.9 | 9.31 | 3522 | 5 |
| 26 | 1,10-bis(4-formylphenoxy)-n-decane | 15.9 | 8.87 | 3535 | 5 |
| 27 | 1,12-bis(4-formylphenoxy)-n-dodecane | 17.7 | 1.32 | 3565 | 6 |
| 28 | 1,16-bis(4-formylphenoxy)-n-hexadecane | 20.4 | 4.05 | 3625 | 9 |
| 29 | 1,2-bis(2-(4-formylphenoxy)ethoxy)ethane | 10.2 | 6.28 | 3511 | 4 |
| 30 | 1,3-bis(3-(p-formylphenoxy)-n-propyloxy)benzene | 15.2 | 22.96 | 3589 | 5 |
| 31 | 1,4-bis(2-(p-formylphenoxy)ethoxy)carbonylbenzene | 11.7 | 24.94 | 3615 | 5 |
| 32 | 5-phenyl-1,3-bis(3-(p-formylphenoxy)-n-propyloxy)benzene | 16.8 | 12.88 | 3664 | 5 |
| 33 | 1,6-bis(4-(4-formylphenyl)phenoxy)hexane | 16.2 | 11.58 | 3633 | 5 |
| 34 | 1,3-bis(5-(4-formylphenoxy)-n-pentyloxy)benzene | 17.04 | 9.31 | 3645 | 6 |
| 35 | 1,8-bis(2-phenyl-5-formylphenoxy)octane | 18.3 | 10.83 | 3662 | 6 |
| 36 | 1,8-bis(3-formylphenoxy)-n-hexane | 15.3 | 2.1 | 3221 | 4 |
| 37 | 1,6-bis(4-(4'-formylphenoxy)phenoxy)-n-hexane | 18.2 | 6.1 | 3347 | 6 |
| 38 | 1,8-bis(3-formyl-2-iodophenoxy)-n-hexane | 22.9 | 2.2 | 3471 | 3 |
| 39 | 1,8-bis(2-phenyl-5-formylphenoxy)-n-octane | 19.3 | 2.8 | 3374 | 4 |
| 40 | 1,3-bis(6-(2-dimethyl)-1-hexanaloxy)benzene | 15.5 | 8.2 | 3229 | 4 |
| 41 | 1,4-bis(6-(2-dimethyl)-1-hexanaloxy)butane | 13.5 | 6.1 | 3209 | 4 |
| 42 | 1,12-bis(4-formylphenoxy)-n-dodecane | 21.6 | 6.8 | 3278 | 5 |
| 43 | 1,3-bis(3-(p-formylphenoxy-n-propyloxy)benzene | HCL SALT | | | |
| 44 | 1,3-bis(3-(p-formylphenoxy-n-propyloxy)-5-n-pentylbenzene | 36.6 | 12.7 | 3660 | 8 |
| 45 | 1,8-bis(3-formyl-2-iodophenoxy)-n-octane | 17.1 | 5.4 | 3762 | 6 |
| 46 | 1,3-bis(6-(2-dimethyl)-1-hexanaloxy)benzene | 13.3 | 15.5 | 3516 | 5 |
| 47 | 1,3-bis(3-(p-formylphenoxy-n-heptyloxy)benzene | 19.3 | 1.4 | 3701 | 6 |
| 48 | 1,4-bis(6-(2-dimethyl)-1-hexanaloxy)butane | 13.5 | 24.8 | 3495 | 4 |
| 49 | 1,3-bis(3-(p-formylphenoxy-n-propyloxy)benzene | HCL SALT | | | |
| 50 | 1,3-bis(3-(p-formylphenoxy)-n-propyloxy)benzene | 13.7 | 12.8 | 3674 | 6 |
| 51 | 1,3-bis(3-(p-formylphenoxy)-n-propyloxy)benzene | 13.2 | 13.1 | 3761 | 9 |
| 52 | 1,3-bis(3-(p-formylphenoxy)-n-propyloxy)benzene | 15.1 | 7.0 | 3333 | 4 |

| | | | | | |
|---|---|---|---|---|---|
| * Conditions A | | | | | |

The present glycopeptide dimers are useful for the treatment of bacterial infections. Therefore, in another embodiment, the present invention is directed to a method for controlling a bacterial infection in a host animal, typically a warm-blooded animal, which comprises administering to the host animal an effective, antibacterial amount of a glycopeptide dimer in which two glycopeptide units are covalently linked to one another through an amine function of an amino sugar. In this embodiment, the dimers can be used to control and treat infections due to various bacteria, but especially gram-positive bacteria. In a preferred embodiment, the dimers are used to control and treat infections due to bacteria resistant to existing antibacterials. For example, certain bacteria are resistant to methicillin, and yet others are resistant to vancomycin and/or teicoplanin. The present dimers provide a technique for controlling and treating infections due to such resistant bacterial species.

In carrying out this embodiment of the invention, the dimers can be administered by any of the conventional techniques, including the oral route and parenteral routes such as intravenous and intramuscular. The amount of compound to be employed is not critical and will vary depending on the particular compound employed, the route of administration, the severity of the infection, the interval between dosings, and other factors known to those skilled in the art. In general, a dose of from about 0.5 to about 100 mg/kg will be effective; and in many situations, lesser doses of from about 0.5 to about 50 mg/kg will be effective. A compound of the present invention can be administered in a single dose, but in the known manner of antibacterial therapy, a compound of the present invention is typically administered repeatedly over a period of time, such as a matter of days or weeks, to ensure control of the bacterial infection.

Also in accordance with known antibacterial therapy, a dimer of the present invention is typically formulated for convenient delivery of the requisite dose. Therefore, in another embodiment, the present invention is directed to a pharmaceutical formulation comprising a dimer of Formula I, in combination with a pharmaceutically-acceptable carrier. Such carriers are well known for both oral and parenteral routes of delivery. In general, a formulation will comprise a dimer in a concentration of from about 0.1 to about 90% by weight, and often from about 1.0 to about 3%.

The antibacterial efficacy of the present dimers is illustrated by following TABLES 3 and 4. The minimal inhibitory concentrations (MICs) were determined using a standard broth micro-dilution assay. TABLE 4 presents a comparison of the activity of illustrative compounds against representative vancomycin-resistant and vancomycin-sensitive enterococci (Enterococcus faecium and Enterococcus faecalis, mean geometric MIC (mcg/mL), as determined by the standard broth micro-dilution assay.

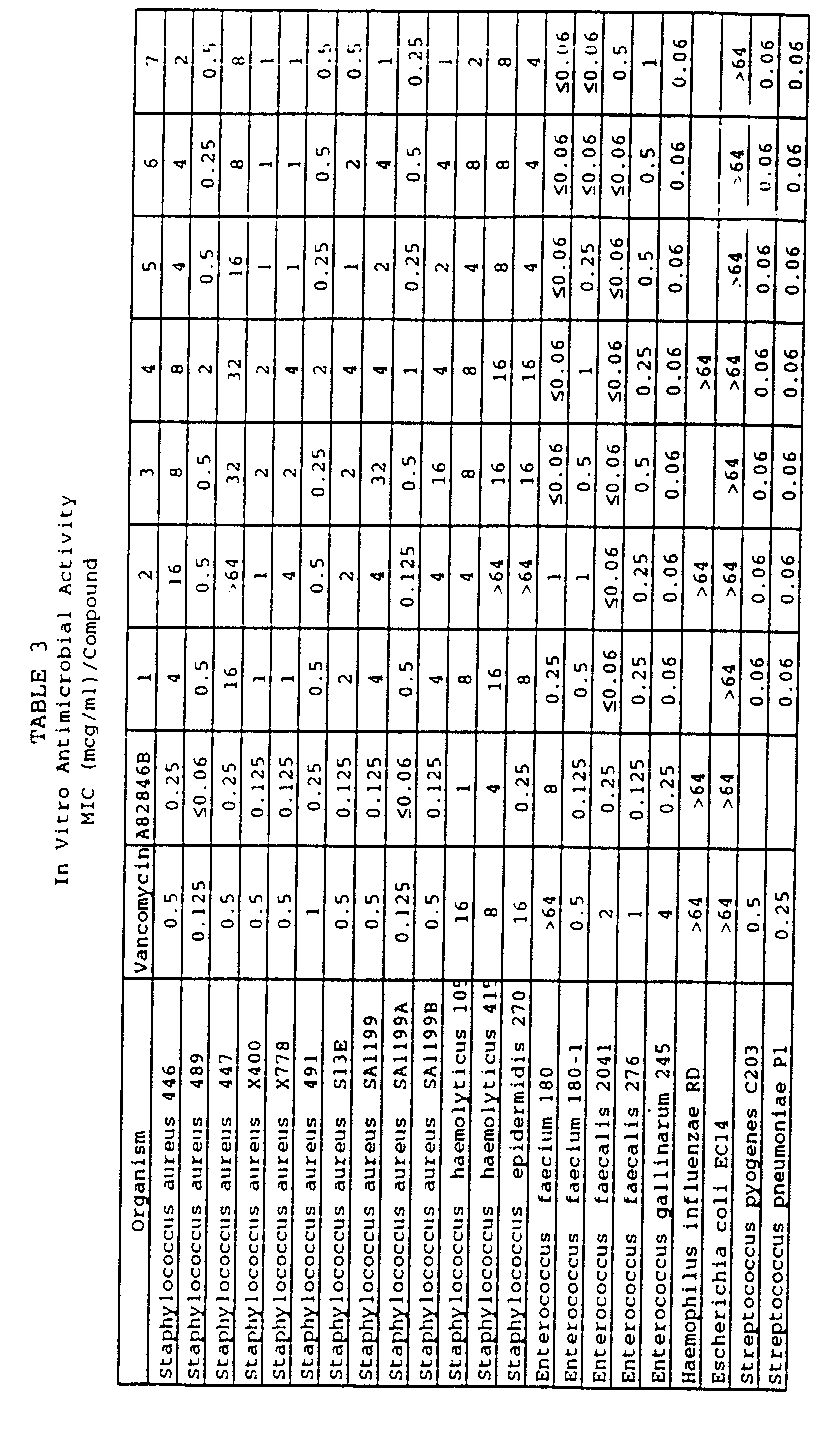

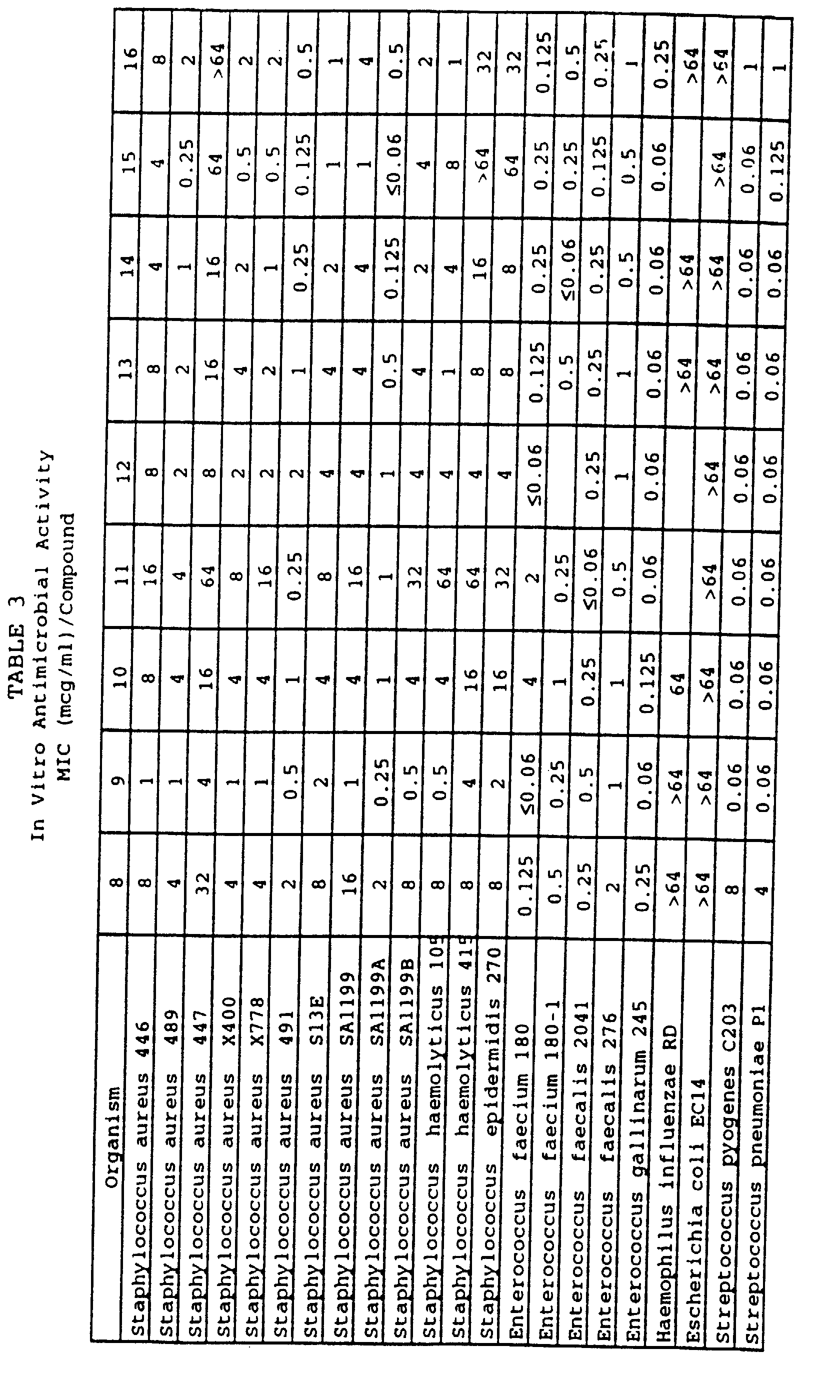

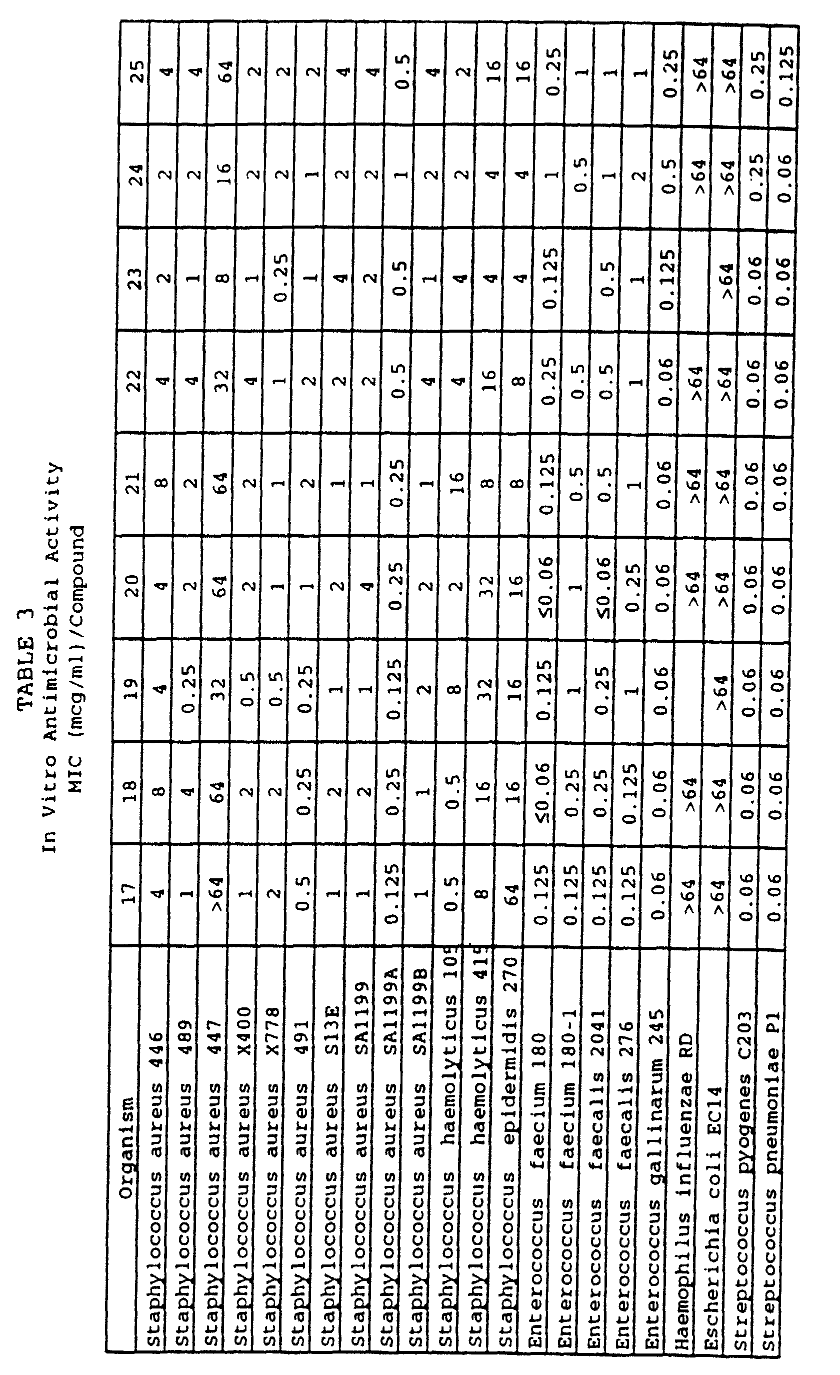

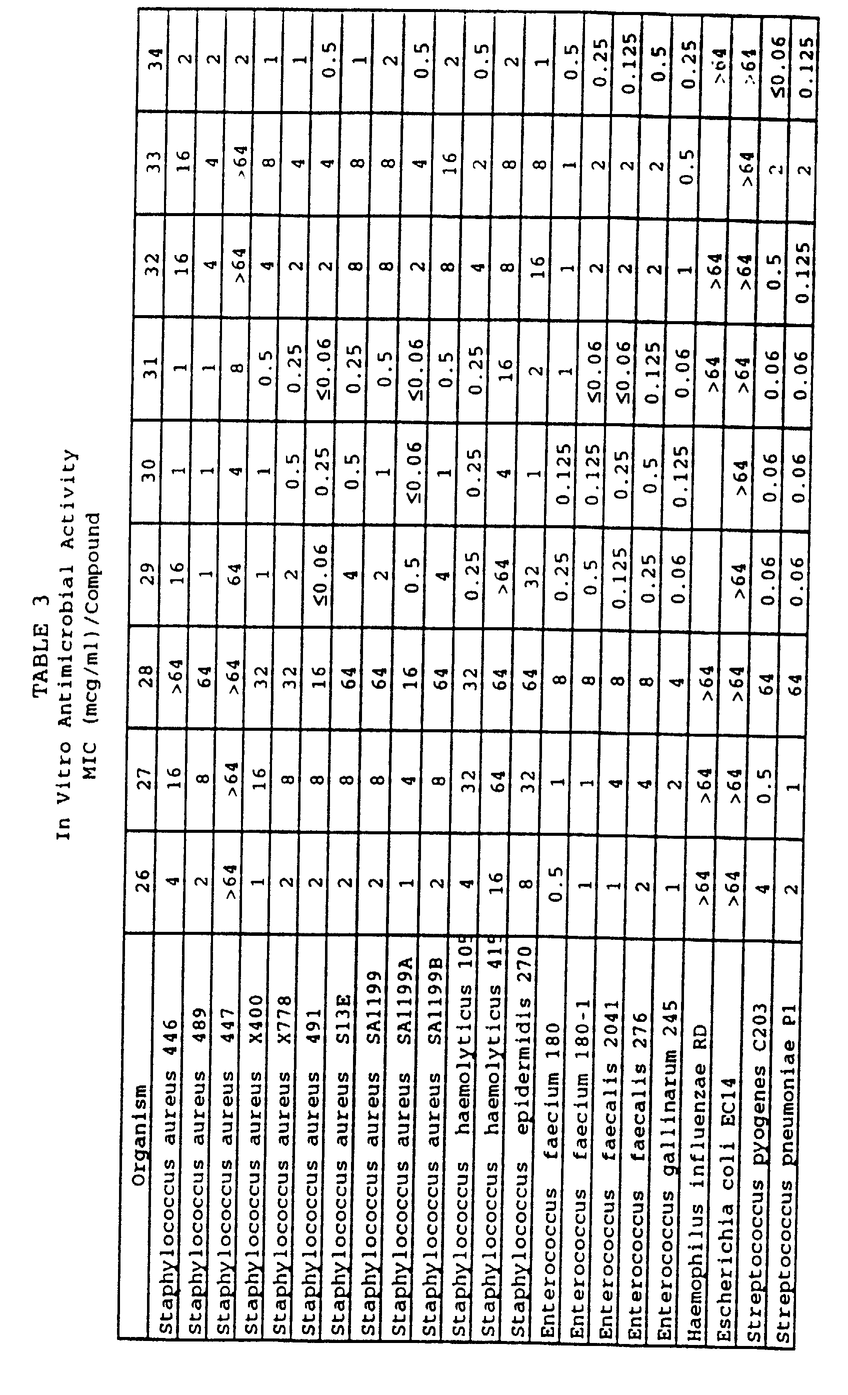

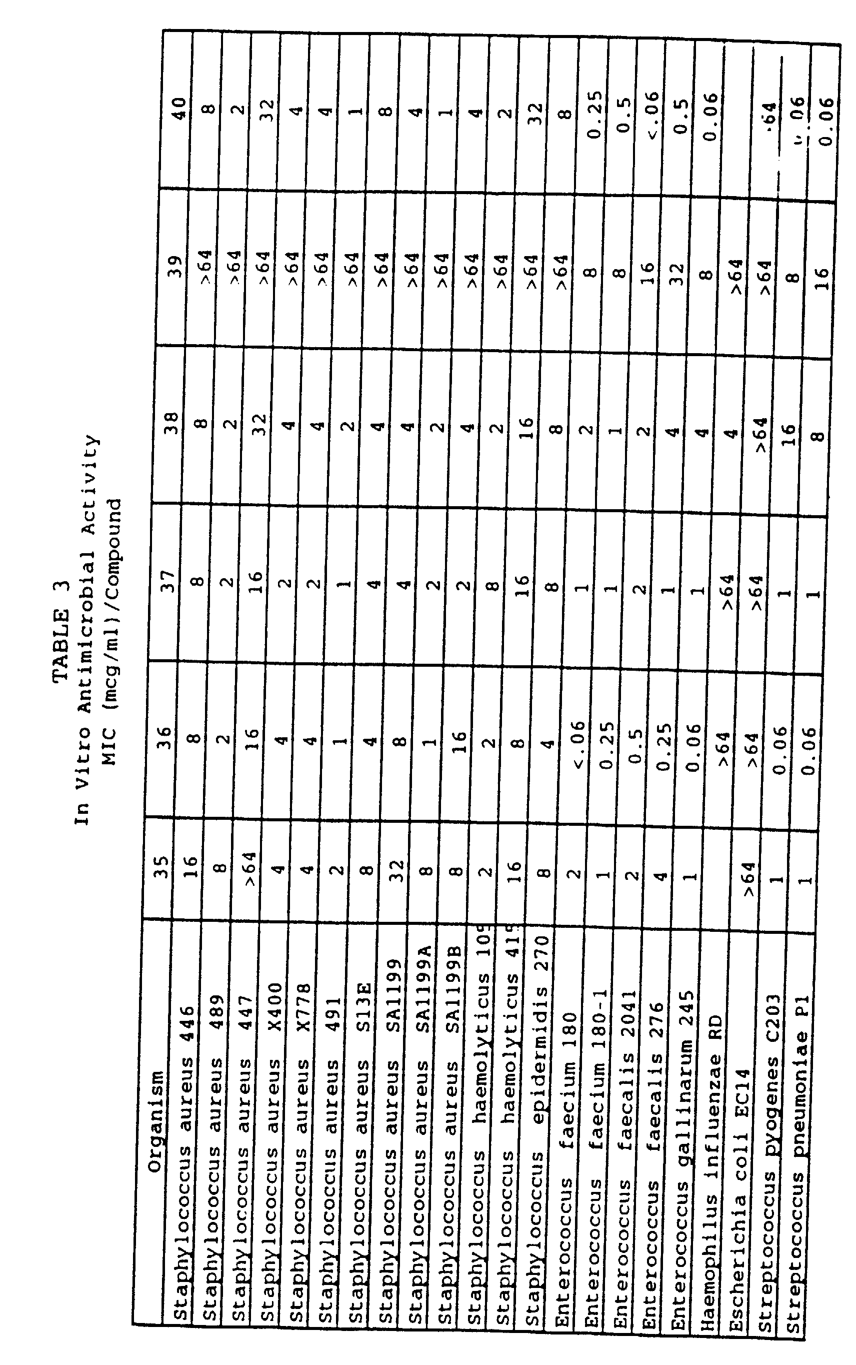

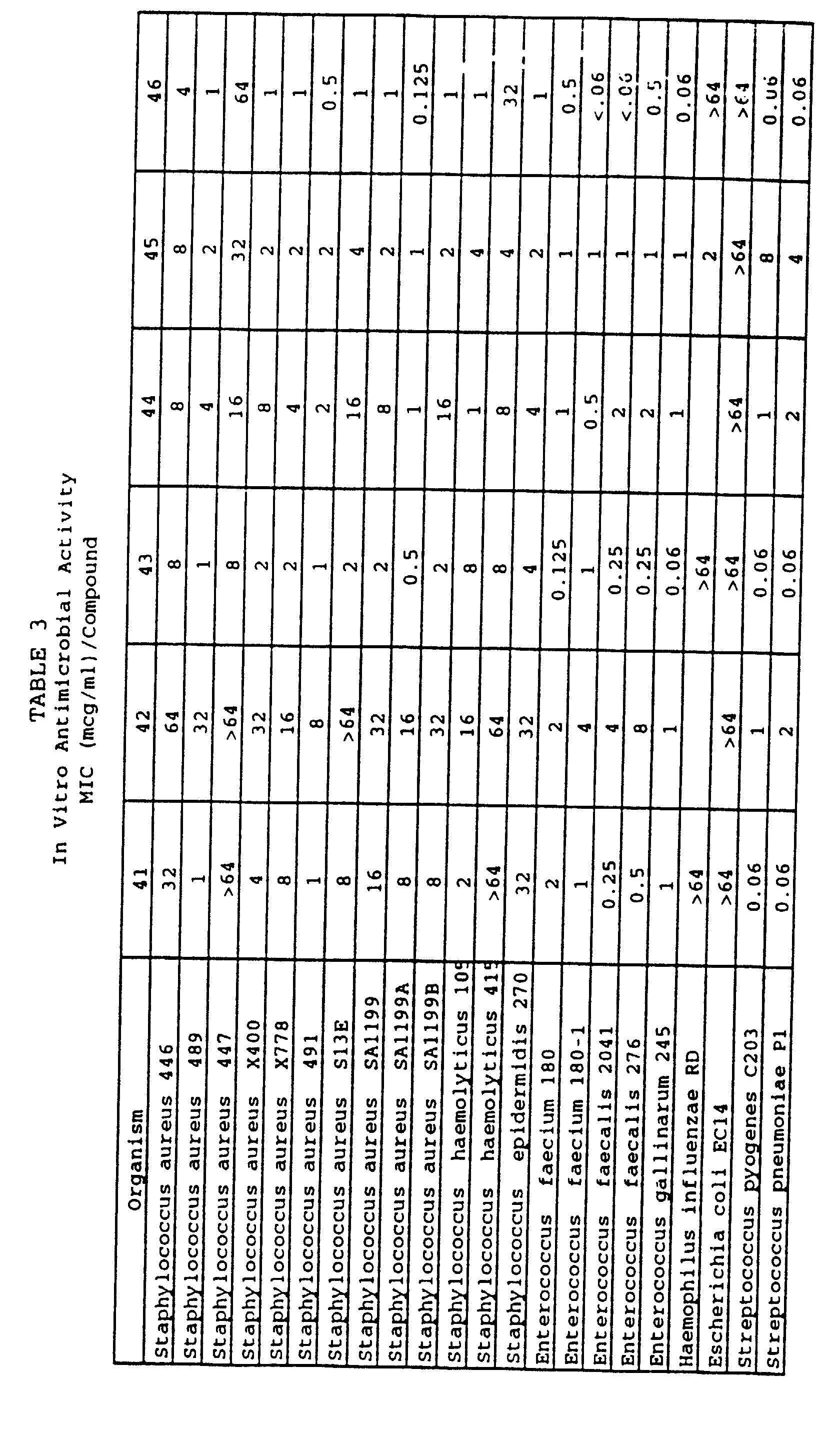

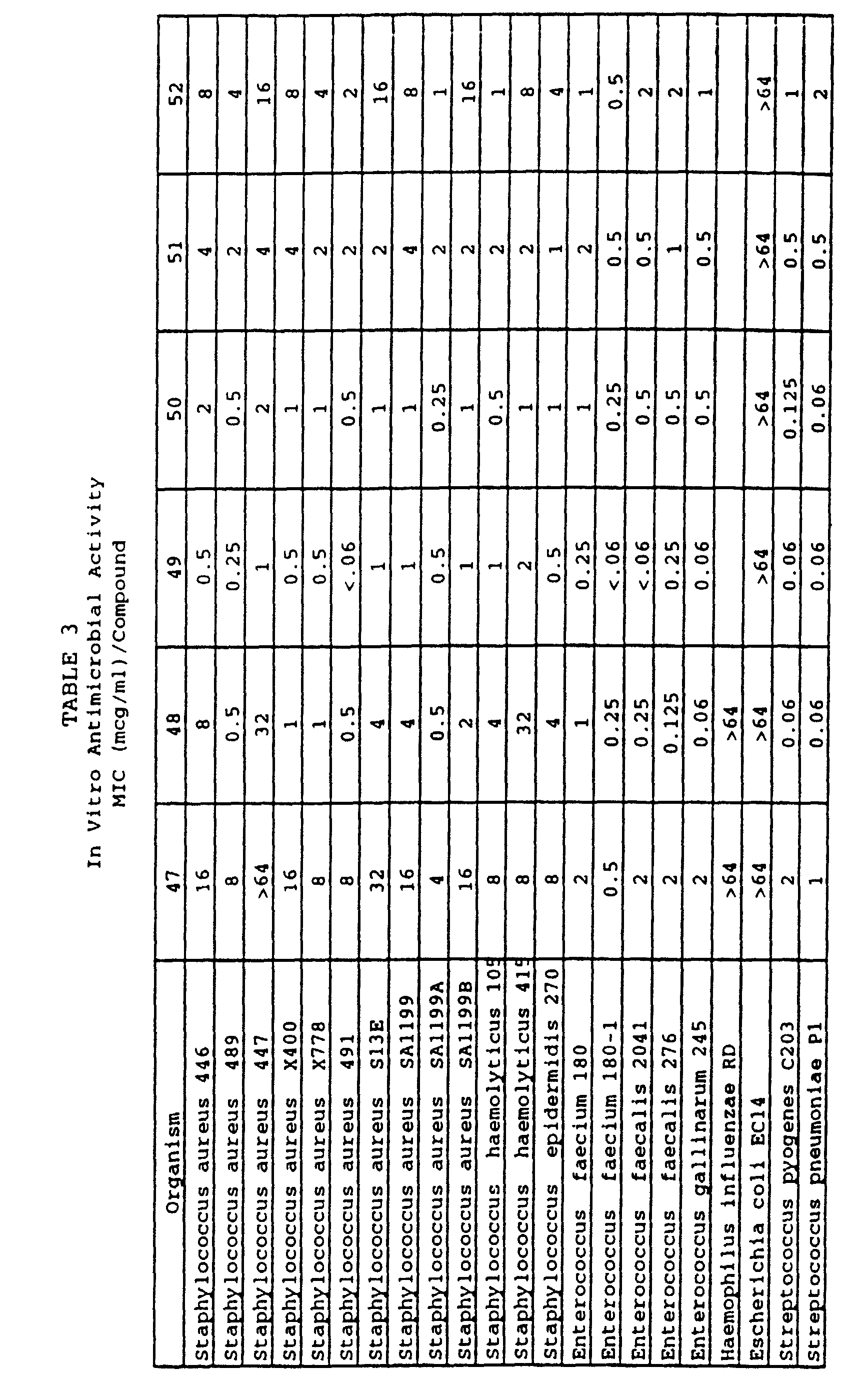

**TABLE 4**

| **In Vitro Activity Against Enterococci** | | |
|---|---|---|
| cpd. Number | Vancomycin Resistant Strains | Vancomycin Sensitive Strains |
| Vancomycin | 282 | 3.9 |
| A82846B | 29 | 0.22 |
| 1 | 42 | 1.3 |
| 2 | 27 | 1.0 |
| 3 | 27 | 1.5 |
| 4 | 19 | 2.0 |
| 5 | 11 | 0.87 |
| 6 | 32 | 1.3 |
| 7 | 8.0 | 1.3 |
| 8 | 9.5 | 0.87 |
| 9 | 9.5 | 1.2 |
| 10 | >90 | 3.0 |
| 11 | 38 | 1.7 |
| 12 | 4.0 | 0.66 |
| 13 | 9.5 | 1.2 |
| 14 | >64 | 1.0 |
| 15 | 23 | 0.87 |
| 16 | 38 | 1.5 |
| 17 | 4.8 | 0.57 |
| 18 | 19 | 1.0 |
| 19 | 19 | 0.76 |
| 20 | 13 | 1.0 |
| 21 | 2.8 | 0.87 |
| 22 | 6.7 | 0.76 |
| 23 | 1.7 | 0.5 |
| 24 | 4.8 | 1.2 |
| 25 | 6.7 | 1.2 |
| 26 | 4.0 | 1.5 |
| 27 | 3.4 | 1.7 |
| 28 | 9.5 | 6.1 |
| 29 | 38 | 1.3 |
| 30 | 1.7 | 0.38 |
| 31 | 27 | 0.66 |
| 32 | 2.8 | 1.5 |
| 33 | 2.0 | 1.5 |
| 34 | 1.7 | 0.44 |
| 35 | 3.4 | 2.3 |
| 36 | 5.7 | 0.66 |
| 37 | 4.8 | 2.3 |
| 38 | 4.8 | 2.3 |
| 39 | 8 | 5.3 |
| 40 | 11.3 | 0.76 |
| 41 | 64 | 1 |
| 42 | 2.4 | 1.2 |
| 43 | 2.4 | 1.3 |
| 44 | 2 | 1.2 |
| 45 | 3.4 | 1.5 |
| 46 | 4.8 | 0.57 |
| 47 | 6.7 | 4 |
| 48 | 22 | 0.57 |
| 49 | 2 | 0.38 |
| 50 | 3.4 | 0.38 |
| 51 | 2.4 | 0.38 |
| 52 | 128 | 4.6 |

## Claims

1. A glycopeptide dimer in which two glycopeptide units are covalently linked to one another through an amine function of an amino sugar, for use in antibacterial therapy.

2. A glycopeptide dimer in which two glycopeptide units are covalently linked to one another through an amine function of an amino sugar, for use in antibacterial therapy against vancomycin-resistant-enterococcus.

3. Use of a glycopeptide dimer according to either of Claims 1 and 2 in the manufacture of a medicament for treating a bacterial infection in a host.

4. Use according to Claim 3 in which both glycopeptide units of the dimer are A82846B.

5. Use according to Claim 3 in which both glycopeptide units of the dimer are vancomycin.

## Patentansprüche

1. Glykopeptiddimer, worin zwei Glykopeptideinheiten kovalent aneinander über eine Aminfunktion eines Aminozuckers gebunden sind, zur Verwendung in der antibakteriellen Therapie.

2. Glykopeptiddimer, worin zwei Glykopeptideinheiten kovalent aneinander über eine Aminfunktion eines Aminozuckers gebunden sind, zur Verwendung in der antibakteriellen Therapie gegen Vancomycin-resistente Enterokokken.

3. Verwendung eines Glykopeptiddimers nach einem der Ansprüche 1 und 2 zur Herstellung eines Arzneimittels zur Behandlung einer bakteriellen Infektion in einem Wirt.

4. Verwendung nach Anspruch 3, worin beide Glycopeptideinheiten des Dimers A82846B sind.

5. Verwendung nach Anspruch 3, worin beide Glycopeptideinheiten des Dimers Vancomycin sind.

## Revendications

1. Dimére glycopeptidique dans lequel deux unités glycopeptidiques sont liées par covalence l'une à l'autre par l'intermédiaire d'une fonction amine d'un sucre aminé, pour l'utilisation en thérapeutique antibactérienne.

2. Dimére glycopeptidique dans lequel deux unités glycopeptidiques sont liées par covalence l'une à l'autre par l'intermédiaire d'une fonction amine d'un sucre aminé, pour l'utilisation en thérapeutique antibactérienne vis-à-vis d'entérocoques résistants à la vancomycine.

3. Utilisation d'un dimére glycopeptidique selon l'une quelconque des revendications 1 et 2 dans la préparation d'un médicament pour traiter une infection bactérienne dans un hôte.

4. Utilisation selon la revendication 3, dans laquelle les deux unités glycopeptidiques du dimére sont le A82846B.

5. Utilisation selon la revendication 3, dans laquelle les deux unités glycopeptidiques du dimére sont la vancomycine.
